# EUROPEAN PATENT APPLICATION

(11) **EP 1 757 244 A1**
(43) Date of publication of application: **28.02.2007**
(21) Application number: 05749027.8
(22) Date of filing: 09.06.2005
(51) Int. Cl.: A61B 18/00, A61B 8/08, A61B 17/22, A61F 7/00, G06T 1/00, H04R 17/00

(54) **ULTRASONIC CURING DEVICE**

(30) Priority: 11.06.2004 JP 2004174214
(71) Applicant: HITACHI MEDICAL CORPORATION, Tokyo 101-0047 (JP)
(72) Inventor: ISHIDA, Kazunari, 2770813 (JP); KUBOTA, Jun, 2700176 (JP); BABA, Hirotaka, 2770005 (JP)
(74) Representative: Brandis -Freiherr von-, Henning
(86) International application number: PCT/JP2005/010613
(87) International publication number: WO 2005/120373

(57) **Abstract**

An ultrasonic treatment apparatus includes a trace and follow computing unit 22 which sets, prior to ultrasonic treatment, a treatment region 52 on an ultrasonic tomographic image 50 of a region of interest containing an affected part of a subject displayed on a monitor 18 as well as sets a reference point 54 outside the treatment region 52, traces a position of the reference point 54 based on image data of the ultrasonic tomographic images taken successively thereafter, computes and estimates moved treatment region 52 due to such as body motion of the subject based on the moved position 54' of the reference point traced and successively outputs a command for correcting a focal point position of treatment use ultrasonic beams irradiated toward the treatment region 52' of the subject from a treatment use probe 24 so as to follow the moved treatment region 52' as computed and estimated. With the trace and follow computing unit 22, the position of the treatment region 52 is each time accurately computed and estimated and based on the computation and estimation the position of the focal point of the treatment use ultrasonic beams follows in real time the movement of the treatment region and is corrected.

## Description

### FIELD OF THE INVENTION

The present invention relates to an ultrasonic treatment apparatus, and in particular relates to an ultrasonic treatment apparatus suitable for irradiating ultrasonic beams correctly toward a treatment region of a subject even if the treatment region moves due to body motion of the subject.

### CONVENTIONAL ART

An ultrasonic treatment apparatus is one in which through transmitting and receiving diagnostic use ultrasonic beams to and from a region of interest of a subject ultrasonic tomographic images (herein below will be indicated as tomographic images) are successively taken, treatment use ultrasonic beams are irradiated while matching the focal point thereof to the treatment region of an affected part (for example, a tumor) with reference to the tomographic image taken and treats the affected part by the energy (for example, heat) of the irradiated treatment use ultrasonic beams.
In such ultrasonic treatment apparatus, in order to denature (for example, to coagulate and necrose) of a biological tissue of the treatment region, the treatment use ultrasonic beams are irradiated continuously to the treatment region for several seconds (for example, 2-10 seconds). Further, in order to denature the biological tissue over the entire affected part, the entire affected part is divided into a plurality of treatment regions, and the treatment use ultrasonic beams are irradiated in a predetermined order to the plurality of the divided treatment regions. Still further, in view of an influence to a healthy biological tissue around the affected part, an interval between the irradiations of the treatment use ultrasonic beams is set comparatively long. For this reason, the treatment time with the treatment use ultrasonic beams is comparatively prolonged.
When the treatment time is prolonged, the affected part possibly moves during the treatment due to body motion such as breathing of the subject and a difference between the focal point of the treatment use ultrasonic beams and the treatment region can be caused. In order for reducing such difference, as disclosed, for example, in JP-A-5-76538, image data of a tomographic image of the affected part taken before the treatment and the tomographic image of the affected part during the treatment are compared and referenced through template matching process, movement of the affected part is detected based on the consistency degree of the image patterns and treatment use ultrasonic transducers are mechanically moved by such as a piston according to the detected movement of the affected part so as to coincide the focal point to the moved affected part.
However, as disclosed, for example, in JP-A-5-76538, when moving the treatment use ultrasonic transducers mechanically, the movement direction and speed of the treatment use ultrasonic transducers are limited in a predetermined range due to the response characteristic of a driving mechanism therefor. As a result, the focal point of the treatment use ultrasonic beams can not sometimes follow the movement of the treatment region and a difference between the focal point of the treatment use ultrasonic beams and the treatment region is possibly caused.
A task of the present invention is to provide an ultrasonic treatment apparatus which correctly traces a movement of biological tissue in a treatment region of a subject due to such as body motion of the subject and causes the focal point of the treatment use ultrasonic beams to follow the movement of the biological tissue in the treatment region of the subject in real time.

### SUMMARY OF THE INVENTION

An ultrasonic treatment apparatus according to the present invention is characterized in that prior to a treatment an image is taken by a diagnosis use probe, in addition to setting of a treatment region on a tomographic image of a diagnosis region of interest containing an affected part of a subject displayed on a monitor by making use of a pointing device such as a mouse, a reference point for specifying the position of the treatment region including a reference region having an area is set at any position outside the treatment region, based on the image data of the tomographic image and image data of a tomographic image of the diagnosis region of interest containing the affected part of the subject which is taken likely by the diagnosis use probe during treatment successively after the setting of the reference point and is displayed on the monitor, a movement of the set reference point is traced, based on the moved position of the traced reference point a moved position of the treatment region of the subject is computed and estimated, and the focal point of the treatment use ultrasonic beams to be irradiated from a treatment use probe is corrected every time so as to follow the computed and estimated moved position of the treatment region of the subject.
More specifically, the ultrasonic treatment apparatus according to the present invention which is provided with a treatment use probe which irradiates treatment use ultrasonic beams to a treatment region of a subject, means for feeding drive signals to the treatment use probe for irradiating treatment use ultrasonic beams, means for successively taking tomographic images through transmitting and receiving diagnosis use ultrasonic beams to and from the subject and a display portion which displays the tomographic images taken, is characterized by comprising a trace and follow computing means which traces a movement such as due to body motion of at least one reference point set on a tomographic image on the display portion for specifying the treatment region of the subject based on image data of the tomographic images taken successively, computes and estimates a movement position of the treatment region based on the movement of the traced reference point and outputs a command for correcting the focal point of the treatment use ultrasonic beams so as to match with the moved position of the treatment region.
According to the present invention, after determining the coordinate position of the moved treatment region based on the movement of the reference point, only by changing delay amounts of ultrasonic drive signals fed to the respective ultrasonic transducers in the treatment use probe so that the treatment use ultrasonic beams are deflected and irradiated toward the determined coordinate position of the treatment region, the focal point of the treatment use ultrasonic beams can be corrected and follow in real time the movement of the treatment region.
If the setting is limited only to the treatment region as in the conventional ultrasonic treatment apparatus, when a biological tissue of the treatment region is denatured by the treatment use ultrasonic beams and the tissue characteristic changes, the image data such as brightness of the treatment region change and a movement of the treatment region due the body motion sometimes can not be detected accurately based on the image data. Accordingly, in the present invention, a reference point for specifying the treatment region is set at the outside of the treatment region, for example, at a position away from the treatment region by a predetermined distance. Thereby, even if the image data of the treatment region have changed by the treatment use ultrasonic beams, since the reference point is set away from the treatment region, the image data of the reference point are hardly affected by the treatment use ultrasonic beams, thus, the movement of the set treatment region can be accurately traced based on the unchanged image data.
Further, according to the present invention, when an amount of movement of a certain reference point exceeds a set amount or when respective movement directions and amounts of movements of a plurality of reference points set are different beyond a set range, such is judged as an erroneous detection because a movement due to body motion is usually in a limited range, thus a command for stopping the irradiation of the treatment use ultrasonic beams is outputted. Accordingly, when the movement of the reference point is erroneously detected, since the irradiation of the treatment use ultrasonic beams is automatically stopped, a possible irradiation by the treatment use ultrasonic beams of a healthy biological tissue can be avoided as well as safety of the apparatus is enhanced. Still further, after the automatic irradiation stop, when a predetermined condition is satisfied, the irradiation is restarted.

### BRIEF DESCRIPTION OF THE DRAWINGS

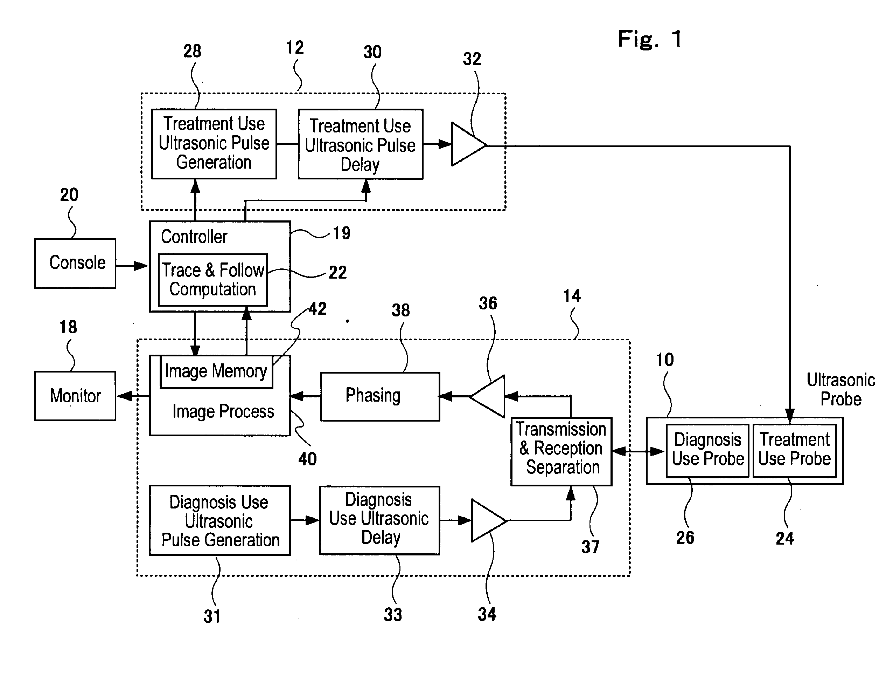

Fig.1 is a block diagram of an embodiment of an ultrasonic treatment apparatus according to the present invention.

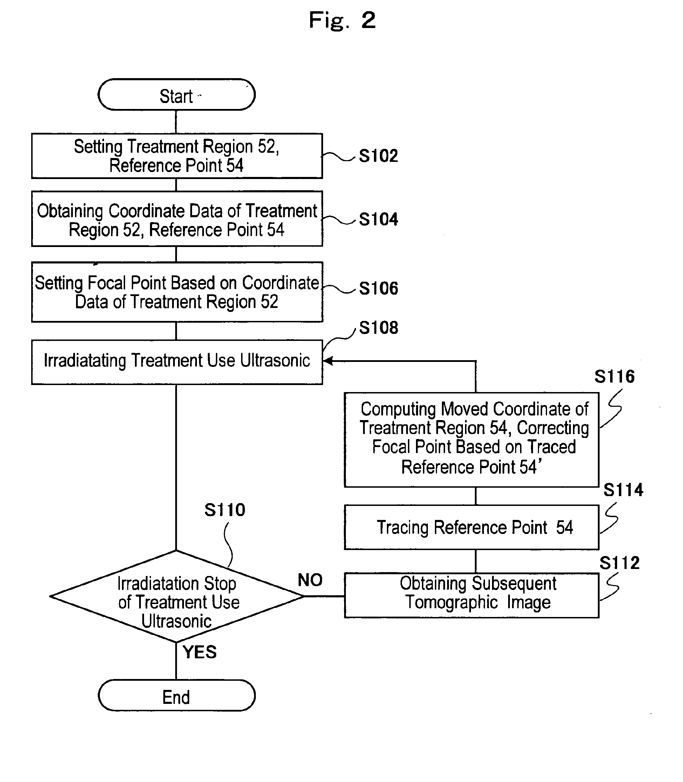

Fig. 2 is a flowchart for explaining an ultrasonic treatment sequence by the ultrasonic treatment apparatus according to the present invention.

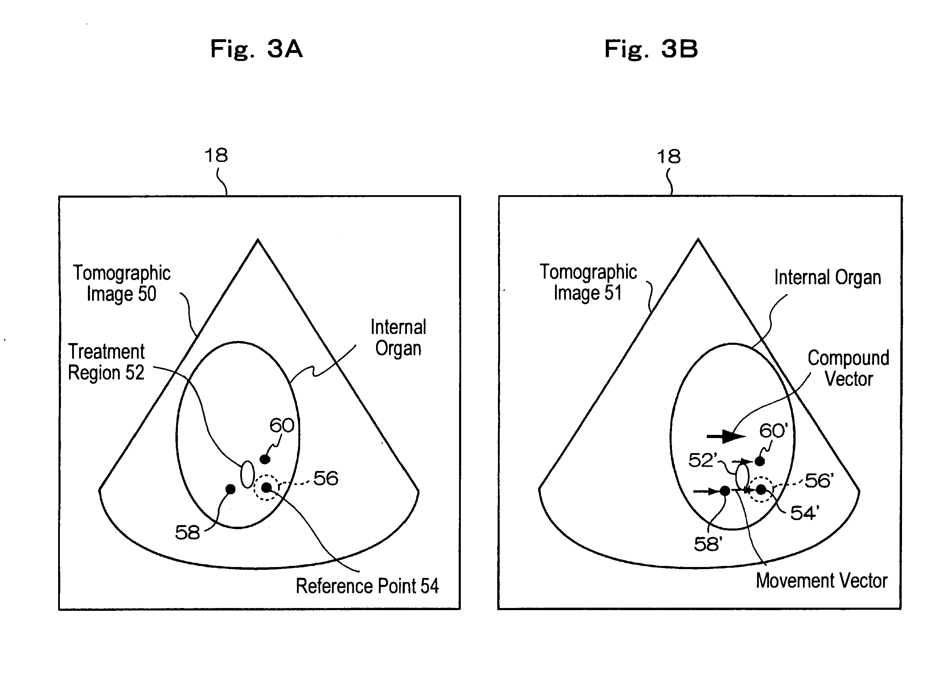

Fig. 3A is a schematic view of an ultrasonic tomographic image of a region of interest containing an affected part of a subject displayed prior to ultrasonic treatment on a monitor of the ultrasonic treatment apparatus according to the present invention and a view for explaining a treatment region of the subject and a reference point for specifying the treatment region both set on the tomographic image according to the present invention.
Fig. 3B is a schematic view of an ultrasonic tomographic image of the same region of interest containing the affected part of the subject displayed at a moment during ultrasonic treatment on the monitor of the ultrasonic treatment apparatus according to the present invention and a view for explaining a movement such as due to body motion of the treatment region and the reference point set previously.

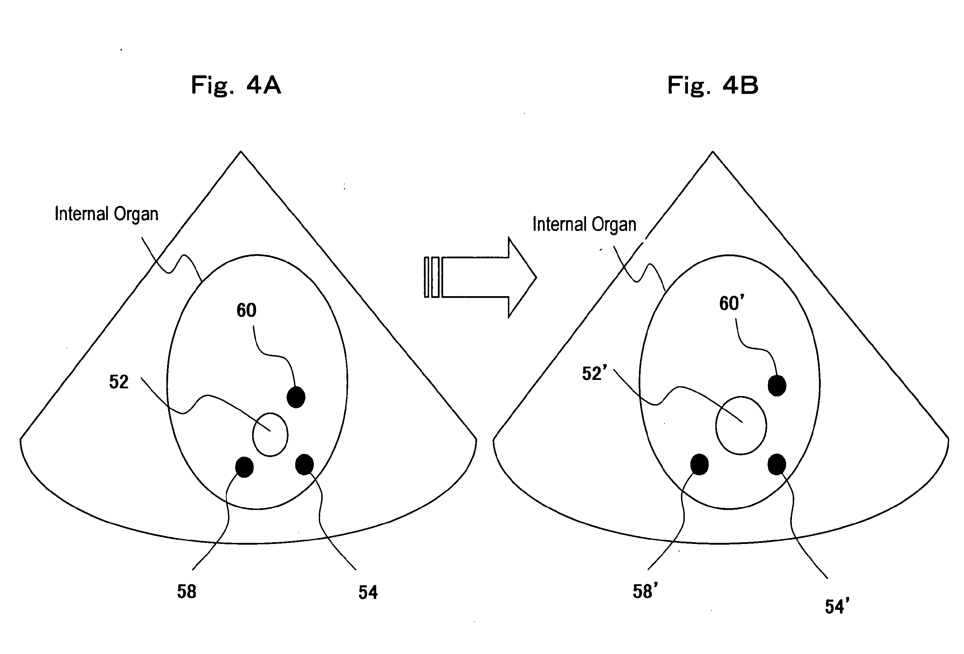

Fig. 4A and 4B are schematic views of ultrasonic tomographic images of same regions of interest containing an affected part of a subject displayed prior and during ultrasonic treatment on a monitor of the ultrasonic treatment apparatus according to the present invention and views for explaining an alternation of a reference point in accordance with an enlargement of a treatment region in association with advancement of the treatment.

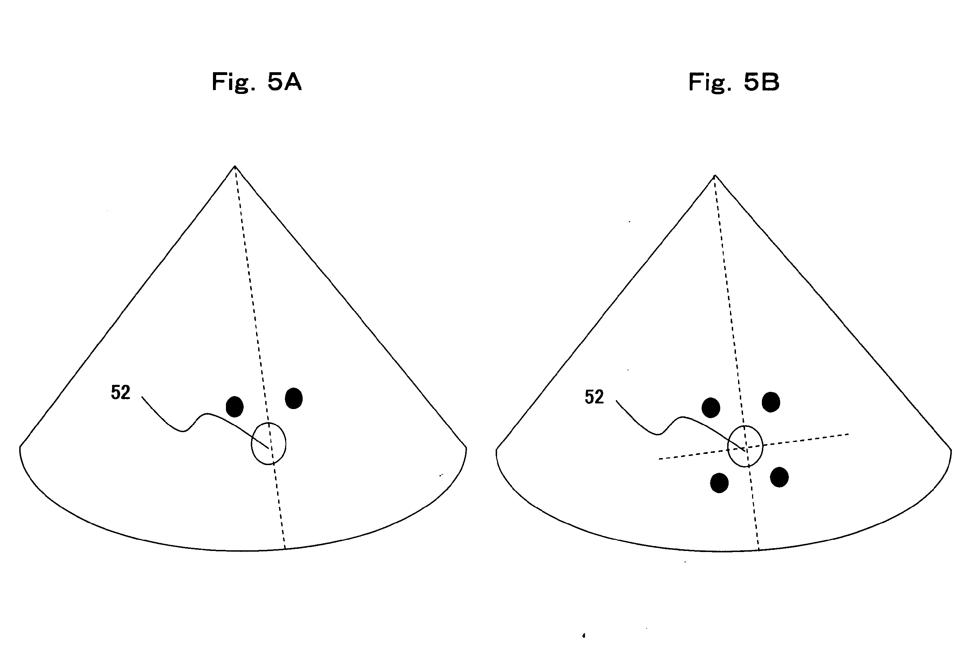

Fig. 5A is a view for explaining an approach when setting one or two reference points on an ultrasonic tomographic image of a region of interest containing an affected part of a subject displayed prior to ultrasonic treatment on a monitor of the ultrasonic treatment apparatus according to the present invention.
Fig. 5B is a view for explaining an approach when setting three or four reference points on an ultrasonic tomographic image of a region of interest containing an affected part of a subject displayed prior to ultrasonic treatment on a monitor of the ultrasonic treatment apparatus according to the present invention.

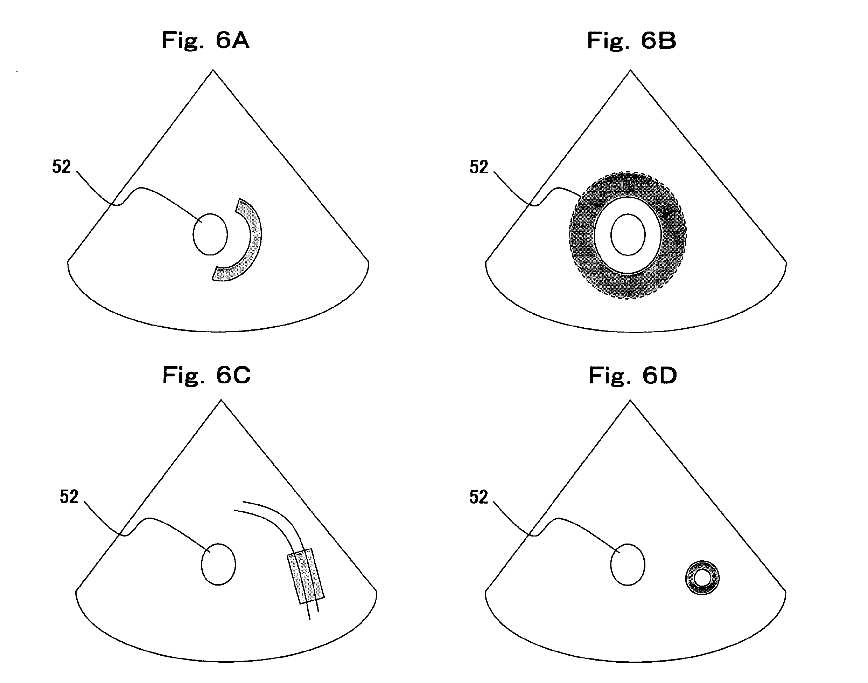

Fig. 6A, 6B, 6C,and 6D are views for explaining a reference region containing one or plural reference points to be set at the outside of a treatment region set on an ultrasonic tomographic image of a region of interest containing an affected part of a subject displayed prior to ultrasonic treatment on a monitor of the ultrasonic treatment apparatus according to the present invention.

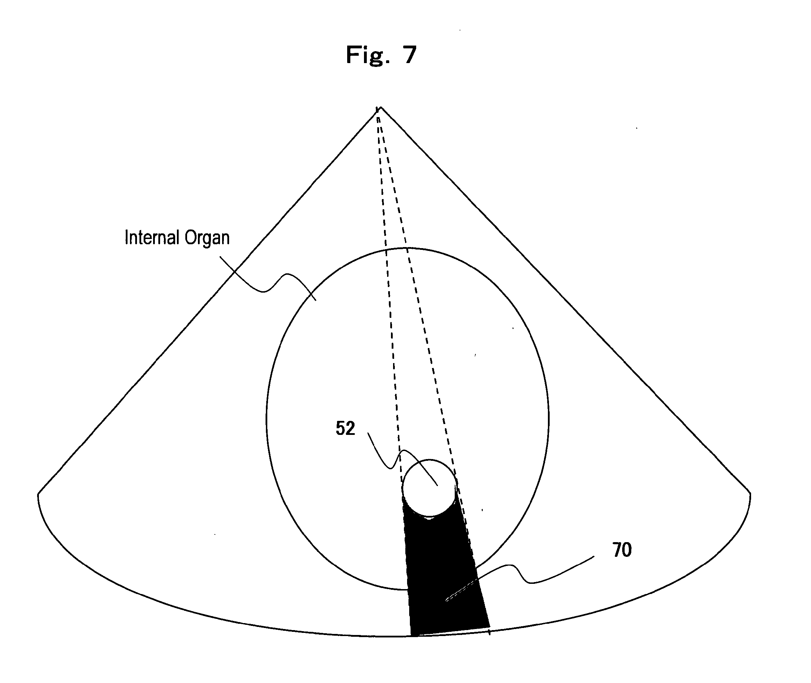

Fig. 7 is an example of ultrasonic tomographic images of a region of interest containing an affected part of a subject displayed during ultrasonic treatment on a monitor of the ultrasonic treatment apparatus according to the present invention and a view for explaining a blackened or a shaded part which appears backward of a treatment region remote from a treatment use ultrasonic probe in association with advancement of the ultrasonic treatment.

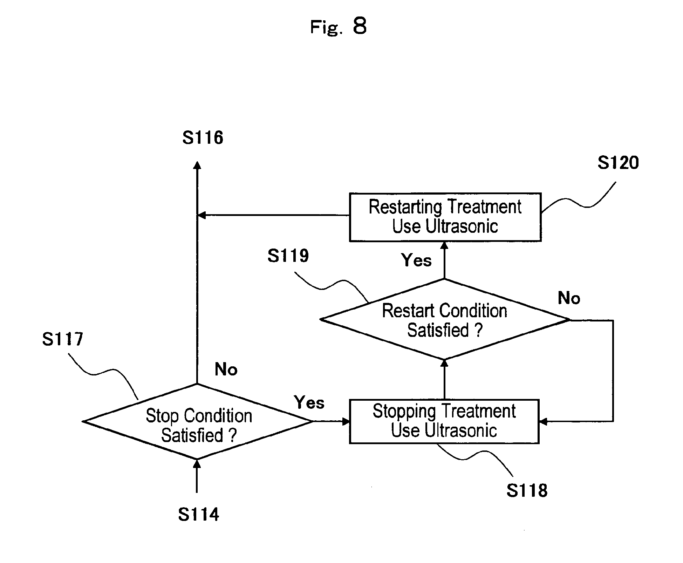

Fig.8 is a flowchart for explaining stopping of irradiation of treatment use ultrasonic beams when the reference point moves beyond a threshold value and restarting of the irradiation of the treatment use ultrasonic beams thereafter when a condition of restarting is satisfied, which is to be added to the flowchart as shown in Fig.2.

### BEST MODES FOR CARRYING OUT THE PRESENT INVENTION

An embodiment of an ultrasonic treatment apparatus to which the present invention is applied will be explained with reference to Fig.1 through 8. Fig.1 is a block diagram of an ultrasonic treatment apparatus to which the present invention is applied. As shown in Fig.1, the ultrasonic treatment apparatus is constituted such as by an ultrasonic probe 10 which irradiates treatment use ultrasonic beams and diagnosis use ultrasonic beams to a subject, a treatment use ultrasonic transmitting portion 12 which feeds treatment use ultrasonic drive signals to the ultrasonic probe 10, a tomographic image taking portion 14 serving as means for feeding diagnosis use ultrasonic drive signals to the ultrasonic probe 10 as well as for successively taking tomographic images based on reflected echo signals received from the ultrasonic probe 10, a monitor 18 serving as means for displaying tomographic images taken successively by the image taking portion 14 and control portion 19 for controlling these respective portions. Further, the control portion 19 is provided with a trace and follow computing portion 22 serving as means for outputting to the treatment use ultrasonic transmitting portion a command for correcting a focal point of the treatment use ultrasonic beams in such a manner that a movement of at least one reference point 54,58,60, which is set for specifying a treatment region 52 set via a console 20 on a tomographic image 50 as shown in Fig.3A containing an internal organ and its affected part of a region of interest and displayed on the monitor 18 prior to the ultrasonic treatment, is traced based on image data of tomographic images taken successively thereafter during the ultrasonic treatment, the position of the treatment region according to the traced movement is computed and estimated and the focal point of the treatment use ultrasonic beams follows the moved treatment region computed and estimated. Still further, in the present embodiment although an example wherein the trace and follow computing portion 22 is implemented as a function of the control portion, the present invention is not limited thereto. Moreover, the console 20 includes a pointing device such as a mouse.
The ultrasonic probe 10 is provided with a treatment use probe 24 which is constituted by arranging a plurality of transducers for irradiating treatment use ultrasonic beams to the subject and a diagnosis use probe 26 which is constituted by arranging a plurality of transducers for transmitting and receiving diagnosis use ultrasonic beams to and from the subject. Although the treatment use probe 24 and the diagnosis use probe 26 are formed integral by arranging in one line, both can be formed in a stacked manner. The point is if both are formed in a manner that the relative position of the treatment use probe 24 and the diagnosis use probe 26 can be grasped, it is satisfactory. Further, although the treatment use probe 24 and the diagnosis use probe 26 in the present embodiment use a convex type in which a plurality of transducers are disposed in one dimensionally along an inner face of a circle, the present invention is not limited thereto and, for example, such as a linear type and a sector type can also be used therefor.
The treatment use ultrasonic transmitting portion 12 is provided with a treatment use ultrasonic pulse generating portion 28 which generates treatment use ultrasonic drive signals, a treatment use ultrasonic delaying portion 30 which applies a focusing process to the ultrasonic drive signals outputted from the treatment use ultrasonic pulse generating portion 28 and an amplifying portion 32 which amplifies the ultrasonic drive signals outputted from the treatment use ultrasonic delaying portion 30 and outputs to the respective transducers in the treatment use probe 24.
The tomographic image taking portion 14 is provided with a diagnosis use ultrasonic pulse generating portion 31 which generates diagnosis use ultrasonic drive signals, a diagnosis use ultrasonic delaying portion 33 which applies a focusing process to the ultrasonic drive signals outputted from the diagnosis use ultrasonic pulse generating portion 31 and an amplifying portion 34 which amplifies the ultrasonic drive signals outputted from the diagnosis use ultrasonic delaying portion 33 and outputs via a transmission and reception separating portion 37 to the respective transducers in the diagnosis use probe 26. The tomographic image taking portion 14 is further provided with another amplifying portion 36 which receives via the transmission and reception separating portion 37 reflected echo signals outputted from the diagnosis use probe 26 and amplifies the same, a phasing portion 38 which phases the phases of the reflected echo signals amplified by the amplified portion 36 and adds the same and an image processing portion 40 which reconstructs tomographic images based on the phased and added reflected echo signals outputted from the phasing portion 38 and outputs the same on the monitor 18. Further, the image processing portion 40 includes an image memory 42, which successively stores reconstructed tomographic images.
An operation of thus constituted ultrasonic treatment apparatus will be explained herein below. At first, the ultrasonic probe 10 is contacted to the body surface or the surface of an internal organ under ventrotomy during an operation. Subsequently, the diagnosis use ultrasonic drive signals are fed to the diagnosis use probe 26 from the image taking portion 14. Through being inputted the fed ultrasonic drive signals to the respective transducers in the diagnosis use probe 26 the ultrasonic beams are transmitted from the respective transducers toward the region of interest of the subject. The echoes reflected from the region of interest of the subject are received by the respective transducers in the diagnosis use probe 26. Two dimensional tomographic images of the region of interest of the subject are reconstructed by the image taking portion 14 based on the received reflected echo signals. The reconstructed tomographic images are stored in the image memory 42. The stored tomographic images, after being read out in response to a control command from the control portion 19, are displayed on the monitor 18, for example, in a fan shape as shown in Fig.3A. Through repeating these processing, a plurality of tomographic images are taken in a predetermined interval and the tomographic images taken are displayed on the monitor 18, for example, 30 frames /second.
Nextly, prior to the ultrasonic treatment, the treatment region 52 and the reference points 54,58 and 60 to be set outside the treatment region for specifying the same are set via the console 20 on the tomographic image 50 displayed on the monitor 18. The focal point of the treatment use ultrasonic beams is determined by the control portion 19 based on the coordinate data of the set treatment region. Further, through outputting a command from the control portion 19 to the treatment use ultrasonic pulse generating portion 28, the treatment use ultrasonic drive signals are produced by the treatment use pulse generating portion 28. The produced ultrasonic drive signals are respectively provided a proper delay by the treatment use ultrasonic delaying portion 30 for every ultrasonic drive signals to be inputted to the respective transducers in the treatment use probe 24 according to the previously determined focal point. Through inputting properly delayed respective ultrasonic drive signals to the respective transducers in the treatment use probe 24, the focal point of the treatment use probe 24 is directed toward the treatment region 52 and ultrasonic beams in a frequency band, for example, 2MHz-4MHz are deflected and irradiated to the treatment region from the treatment use probe 24. By the ultrasonic energy (for example, heat) irradiated the biological tissue in the treatment region is heated and cauterized and the treatment is achieved (for example by necrosing and coagulating). In this manner, the affected part is treated by HIFU (High Intensity Focused-Ultra Sound). Further, the frequency band of the treatment use ultrasonic can be altered properly (for example, 500kHz).
During such ultrasonic treatment, a divergent between the focal point and the treatment region can be sometimes caused because of movement of the affected part due to body motion such as breathing of the subject or displacement of the ultrasonic probe position, which is grasped by a hand of an operator. The trace and follow computing portion 22 of the present embodiment accurately detects such divergence between the focal point of the treatment use ultrasonic beams and the treatment region and corrects the focal point of the treatment use ultrasonic beams so as to follow the same in real time to the movement of the biological tissue in the treatment region.
Herein below, the trace and follow computing portion 22 of the present embodiment will be explained in detail with reference to Figs. 2, 3A and 3B. At first, the treatment region 52 and the reference point 54 are set via the console 20 on the tomographic image 50 displayed on the monitor 18 (S102). For example, the treatment region 52 can be set by designating an extent to be treated on the tomographic image 50 with a mouse. Further, a predetermined extent from a point designated by the mouse can be automatically set as the treatment region 52. The set extent of the treatment region can also be set, for example, according to a volume of the biological tissue which is coagulated and necrosed by the irradiated ultrasonic beams when ultrasonic beams (Therapy beams, herein after will be called T beams) formed by the treatment use ultrasonic beams are irradiated one time for a predetermined time. Then, the reference point 54 is set via the console 20 so as to locate at the outer region from the treatment region 52, for example, at a position away from the treatment region 52 by 5mm.
Further, the image region corresponding to the treatment region 52 can be displayed by superposing a first color marker. In addition to the first color marker, the focal point of the treatment use ultrasonic beams can be displayed by superposing a second color marker. Thereby, a divergence between the treatment region and the focal point can be visibly grasped.
Subsequently, the coordinate data of the treatment region 52 and the reference point 54 on the display are calculated in response to the setting of a pointer of the mouse (S104). The focal point of the treatment use ultrasonic beams is set based on the calculated coordinate data of the treatment region 52 (S106). The treatment use ultrasonic delay portion 30 sets respective delay amounts of the ultrasonic drive signals to be provided to the respective transducers in the treatment use probe 24 so that the treatment use ultrasonic beams reach the set focal point at the same time. The treatment use ultrasonic beams according to the set delay amounts are irradiated from the treatment use probe 24 to the treatment region 52 (S108). The irradiated treatment use ultrasonic beams are focused to the focal point and provide a comparatively strong ultrasonic energy to the treatment region.
When the treatment use ultrasonic beams are irradiated beyond a predetermined set time or when a termination command is input from the console 20, the irradiation of the treatment use ultrasonic beams is stopped (S110). Further, when the treatment use ultrasonic beams are irradiated to the treatment region over the predetermined time, the treatment of the treatment region 52 is estimated having been completed, and if a measure such as changing the first color marker representing the treatment region 52 is taken, the completion of the treatment can be visibly grasped.
During irradiation of the treatment use ultrasonic beams, the trace and follow computing portion 22 successively reads out tomographic images 51 from the image memory 42 (S112).
The reference point 54 is traced based on the image data of the tomographic images 51 successively read out from the image memory 42 and the image data of the previous tomographic image 50 (S114). For example, a region containing the reference point 54 on the tomographic image 50 is carved out. The carved out region, namely, an extent of the reference region is set via the console 20 or automatically at a predetermined extent. Through comparison between the carved out region, namely, the extent of the reference region 56 and the image pattern (for example, brightness distribution pattern) of the tomographic image 51, a same size region showing the highest image coincidence degree with the region 56 is extracted from the tomographic image 51 as a traced region 56'. The coordinate position of the traced region 56' as extracted is obtained as the coordinate position of the moved position of the region 56. A movement vector (for example, movement direction and movement amount) of the reference point 54 is determined by making use of the obtained coordinate position. Through the use of the determined movement vector the moved coordinate of the treatment region 52 (namely, the coordinate position of the treatment region 52') is obtained. Further, as a method of determining the image coincidence degree, such as block matching method and SAD (Sum of Absolute Difference) are applied.
Figs.6A, 6b, 8C and 6D are views showing variation shapes of carved out regions, namely reference regions containing one or plural reference points, Fig.6A exemplifies a strip shaped reference region along the outer circumference of the treatment region 52 and Fig.6B exemplifies a mask region including the treatment region and the vicinity thereof and the reference region surrounding the outer circumference of the mask region, wherein the extent of the outer circumference region indicated by a dotted line is arbitrary and the entire tomographic image other than the mask region can be the reference region. Fig.6C exemplifies a reference region containing at least a part of a blood vessel running in parallel with the tomographic image and Fig.6D exemplifies a reference region containing therein a cross section of a blood vessel running perpendicularly to the tomographic image. As will be understood from the above, the size of a selected reference region containing a reference point is not in particular limited.
The focal point of the treatment use ultrasonic beams is corrected by the trace and follow computing portion 22 based on the coordinate position of the treatment region 52' (S116). The corrected new focal point is inputted to the treatment ultrasonic delaying portion 30 as corrected data and the treatment use ultrasonic beams are deflected and irradiated based on the inputted focal point (S108). Through repeating these processes S108~S116 with a predetermined timing, for example, at the timing when a tomographic image is taken in, the focal point of the treatment use ultrasonic beams is precisely corrected following the movement of the treatment region 52.
According to the present embodiment, since the moved coordinate position of the treatment region 52 can be determined from the movement of the reference point 54, when the moved coordinate position of the treatment region 52 is computed and estimated according to the movement of the reference point 54 and the treatment use ultrasonic beams are deflected and irradiated toward the computed and estimated moved coordinate position, the focal point of the treatment use ultrasonic beams can follow in real time the movement of the treatment region 52. Namely, through monitoring the movement vector of the reference point 54 in real time or at a predetermined timing, the movement of the treatment region 52 is precisely detected and based on the detection the focal point of the treatment use ultrasonic beams can be accurately corrected.
Further, in the present embodiment, the reference point 54 is set at a position outside the treatment region 52 and away from there by a predetermined distance, for example, an extent which is coagulated and necrosed by irradiation of treatment use ultrasonic beams in one time, more specifically, 5mm. Thereby, even when the biological tissue in the treatment region is coagulated and necrosed, since the image data of the region 56 containing the reference point 54 do not change, the movement of the reference point 54 can be precisely traced based on the image data.
It is preferable to set the reference point at a position where no sound rays of the treatment use ultrasonic beams overlap. Further, it is preferable to select a characteristic region of a pixel value distribution pattern in the reference region and to set the reference point at the selected region. For example, as the characteristic region, a region showing a local maximum or a local minimum pixel value distribution and a region containing such as a characteristic internal organ like a blood vessel and a region such as a membrana capsule which will be blackened or shaded because of water accumulation therein are enumerated. Further, when setting the reference point automatically, a selection standard as explained above with a predetermined rule having a priority is stored in the trace and follow computing portion 22 beforehand, thus, every time when a treatment region is set, the reference point is automatically set by the trace and follow computing portion 22.
Further, when the movement amount of the reference point 54 exceeds a set value (threshold value), a command for stopping the irradiation of the treatment use ultrasonic beams can be outputted from the trace and follow computing portion 22 to, for example, the treatment ultrasonic pulse generating portion 28 in the treatment use ultrasonic transmission portion 12. Thereby, when the movement of the reference point 54 is detected to have e4xceeded beyond the threshold value, the irradiation of the treatment use ultrasonic beams is stopped automatically, thus a possible irradiation of the treatment use ultrasonic beams to a healthy biological tissue can be avoided as well as the safety of the apparatus can be enhanced. Further, the set value can be variably set according to circumstances.
Fig.8 is a flowchart to be added between S114 and S116 in Fig.2 flowchart and for explaining the sequence of stopping of the treatment use ultrasonic beams irradiation when the movement of the reference point exceeds the threshold value and of restarting the irradiation thereafter. Namely, when judged at S117 that the condition of stopping the treatment use ultrasonic beams as explained above is satisfied, the irradiation of the treatment use ultrasonic beams is stopped at S118. Subsequently, at S119, it is judged whether the condition of restarting irradiation of the treatment use ultrasonic beams, for example, such as whether the movement amount of the reference is stabilized in a predetermined range for a predetermined time continuously or whether the movement amount of the reference point returns in a predetermined range, is judged, and it is judged that the condition is satisfied, a process returning to a necessary routine for restarting irradiation of the treatment use ultrasonic beams is performed at S120.
Herein above the present invention has been explained with reference to the embodiment, however, the present invention is not limited thereto. For example, since the extent of the treatment region 52 can be grasped beforehand as a burning region of the treatment use ultrasonic beams, the reference region 54 can be set automatically at the outside of the treatment region in stead of manually setting the reference point 54. Thereby, since the reference point 54 is automatically set by simply designating the treatment region 52, an easy-to-use of the apparatus is enhanced.
Further, as shown in Fig.3A, a plurality of reference points such as reference points 58 and 60 in addition to the reference point 54 can be set. In this instance, the moved coordinate position of the treatment region (namely, the coordinate position of the treatment region 52') is obtained based on an average of movement vectors of the respective reference points or a compound vector determined by multiplying a weighted coefficient. In this way, a possible erroneous setting of the focal point due to erroneous detection of movement of the reference point can be greatly reduced.
Figs. 5A and 5B are views for explaining an approach of determining setting position of reference points depending on the number thereof when 1-4 reference points are set near the treatment region 52. Fig.5A shows an example when one or two reference points are set, the tomographic image is divided into two by a sound ray (in dotted line) passing through the center of the treatment region 52, when setting one reference point, the reference point is set at either region, and when two reference points are set, the reference points are set at both regions respectively. Preferably, the reference regions are set at the upper side (at the side near the probe) of the treatment region 52.
Fig.5B shows an example when three or four reference points are set, the tomographic image is divided into four by a sound ray (in dotted line) passing through the center of the treatment region 52 and a straight line perpendicular to the sound ray, the third reference point is set on either region at the lower side (at the side remote from the probe), and when four reference points are set, the third and fourth reference points are set at both lower regions respectively. Preferably, these reference points are set in an equiangle. Further, when setting reference points of more than four, the reference points are set in the same approach. Namely, by using the center of the treatment region as the reference, the treatment region 52 is divided (preferably equally) depending on the number of reference points to be set and the respective reference points are set at the outside of the respective divided regions.
When the irradiation of the treatment use ultrasonic beams to the treatment region advances, the treatment region begins to be coagulated and necrosed, and a blackened portion 70 of low signal region appears in connection with the coagulated and necrosed region at a region opposite to the treatment use probe as shown in Fig.7. This is because the ultrasonic beams are reflected at the boundary of the coagulated and necrosed region and do not penetrate through the coagulated and necrosed region. Accordingly, by making use of this phenomenon the blackened region 70 in the sound ray direction on the tomographic image is detected and traced and the moved position due to such as body motion of a region immediately before the blackened region representing the treatment region is computed in parallel with the computation of the moved position of treatment region based on the trace of the reference point and is combined therewith, thereby, the detection accuracy of the treatment region can be further enhanced.
Further, when a biological tissue is coagulated and necrosed by irradiation of treatment use ultrasonic beams, the tissue is hardened, therefore, when the coagulated and necrosed hardened region is directly detected and traced on a modulus of elasticity image of a biological tissue as disclosed, for example, in JP-A-2000-60853 and the moved position due to such as body motion of the treatment region is computed in parallel with the computation of the moved position of treatment region based on the trace of the reference point and is combined therewith, thereby, the detection accuracy of the treatment region can be further enhanced.
Still further, the computation of the moved position of the treatment region by tracing the reference point can be performed in parallel by combining with both computations of moved position of the region immediately before the blackened region and moved position of the coagulated and necrosed hardened region.
Further, in the explanation of above embodiment, the trace of the reference point 54 is performed by comparing the tomographic image on which the reference point 54 is set prior to the ultrasonic treatment with image data of the tomographic images 51 successively obtained thereafter, however, the reference point 54 can be traced by comparing successive two tomographic images in connection with the initially set reference point 54.
Further, with regard to a treatable extent (for example, volume) when treatment use ultrasonic beams (T beams) are irradiated once, there is a certain limitation (for example, 4~10mm³), therefore, when an affected part extends in broad area (for example, several cm³), the affected part is divided into a plurality of treatment regions and the treatment use ultrasonic beams (T beams) are irradiated to every divided treatment region. In such instance, an irradiation region which is treatable by one time irradiation of the treatment use ultrasonic beams is calculated before hand and the affected part is automatically divided small into a plurality of treatment regions according to the calculated irradiation region of a predetermined extent. Thereby, since such as overlapping of the irradiation regions can be prevented and a gap occurrence between the adjacent irradiation regions can be avoided, irradiation over necessity of the treatment use ultrasonic beams to a biological tissue is prevented and occurrence of omitted treatment portions can be avoided.
Figs.4A and 4B are views for explaining an automatic renewal of reference points set outside a treatment region in association of renewal and expansion of the treatment region. Fig.4A corresponds to Fig.3A. When expanding the treatment region to the subsequent region 52' after the treatment of the treatment region 52 is completed, the reference points 54, 58 and 60 set previously are automatically altered to the reference points 54', 58' and 60' according to a predetermined rule in association with the expansion of the treatment region.
Further, when irradiating the treatment use ultrasonic beams to a same treatment region in a plurality of times, since the energy (for example, heat) of the irradiated treatment use ultrasonic beams possibly causes an adverse influence to a healthy tissue other than an affected part, it is preferable to properly set an irradiation waiting time from the irradiation stop of the treatment use ultrasonic beams to the subsequent irradiation restart. Thereby, since the temperature of the healthy tissue lowers during the lapse of the irradiation waiting time, the adverse influence to the healthy tissue by the energy of the treatment use ultrasonic beams can be reduced.
When setting an irradiation waiting time (irradiation interval) of the treatment use ultrasonic beams, the ultrasonic treatment apparatus of the present invention can be used in combination with a temperature detecting means in such a manner that when the temperature of the healthy tissue other than the affected part is detected to be lowered below a predetermined value by the temperature detecting means, the irradiation of the treatment use ultrasonic beams can be restarted. For example, as the temperature detecting means a magnetic resonance imaging (MRI) device is used, and with the MRI device a temperature distribution image of a cross sectional area containing the treatment region and a healthy tissue around the region is obtained and based on the temperature variation of pixels on the temperature distribution image the irradiation stop and restart can be controlled. Thereby, since the irradiation waiting time is properly and automatically adjusted, the efficiency and safety of the apparatus is enhanced. Herein, in the temperature distribution image, for example, pixels corresponding to a high temperature tissue are shown in red and depending on temperature decrease the color gradually changes from red to blue. Further, since it is preferable that the temperature distribution image is renewed in real time, the spatial resolution of the MRI image is suppressed comparatively low and the repetition time (TR) of RF irradiation pulses in the pulse sequence is shortened according to circumstances. Further, the irradiation stop and restart control of the treatment use ultrasonic beams which is performed by monitoring the temperature variation of the healthy tissue around the affected part with the MRI device is performed in parallel with the irradiation stop and restart control of the treatment use ultrasonic beams which is explained in connection with Fig.8 flowchart and is executed when the movement of the tracing reference point exceeds the threshold value.

## Claims

1. An ultrasonic treatment apparatus comprising:
a treatment use probe which irradiates treatment use ultrasonic beams to a treatment region of a subject; means for feeding drive signals to the treatment use probe for irradiating treatment use ultrasonic beams; means for successively taking ultrasonic tomographic images of a region of interest containing the treatment region of the subject through transmitting and
receiving diagnosis use ultrasonic beams to and from the subject; means for displaying the ultrasonic tomographic images taken; and an inputting means for setting the treatment region on the ultrasonic tomographic image displayed on the displaying means, **characterized in that** the ultrasonic treatment apparatus further comprising a trace and follow computing means which traces a movement such as due to body motion of the subject of at least one reference point set at the outside of the treatment region set via the inputting means on an ultrasonic tomographic image displayed on the display portion and for specifying the treatment region based on image data of the ultrasonic tomographic images taken successively, computes and estimates a movement position of the treatment region according to the traced moved reference point and outputs a command to the drive signal feeding means for altering and correcting delay amounts of respective transducers in the treatment use probe so that the focal point of the treatment use ultrasonic beams irradiated from the treatment use probe follows the moved position computed and estimated of the treatment region.

2. An ultrasonic treatment apparatus according to claim 1, **characterized in that** the correction command of the focal point of the treatment use ultrasonic beams outputted from the trace and follow computing means to the drive signal feeding means is a command for altering and correcting the delay amounts of the respective transducers in the treatment use probe.

3. An ultrasonic treatment apparatus according to claim 1, **characterized in that** the reference point is set at a position which does not overlap irradiation sound rays of the treatment use ultrasonic beams.

4. An ultrasonic treatment apparatus according to claim 1, **characterized in that** the reference point is set at a position away from the treatment region set by a predetermined distance.

5. An ultrasonic treatment apparatus according to claim 1, **characterized in that** the reference point is set at a position where a pixel value distribution pattern of a reference region containing the reference point therein is different from a pixel value distribution pattern of a region outside the reference region.

6. An ultrasonic treatment apparatus according to claim 1, **characterized in that** the reference point is set at least one divided region, each of which is formed by dividing a region outside the treatment region into a plurality of regions with reference to the center of the treatment region.

7. An ultrasonic treatment apparatus according to claim 1, **characterized in that** when setting one reference point, the ultrasonic tomographic image is divided into two by a sound ray passing the center of the treatment region and the one reference point is set at one of the divided regions, when setting two reference points, each is set at one of both regions, when setting three reference points, the ultrasonic tomographic image is divided into four by the sound ray and a straight line perpendicular to the sound ray passing the center of the treatment region and the third reference point is set at either lower side region remote from the treatment probe and when setting four reference points, each of the third and fourth reference points is set at one of lower side both regions.

8. An ultrasonic treatment apparatus according to claim 1, **characterized in that** the reference point is within a region containing at least a part of a blood vessel running in the tomographic image.

9. An ultrasonic treatment apparatus according to any one of claims 3 through 8, **characterized in that** the trace and follow computing means stores a selection rule of the reference point with priority, and when the treatment region is set via the inputting means, the trace and follow computing means automatically set the reference point based on the reference point selection rule stored.

10. An ultrasonic treatment apparatus according to any one of claims 3 through 8, **characterized in that** the trace and follow computing means stores a selection rule of the reference point with priority, and every time when the treatment region set via the inputting means is renewed and expanded, the trace and follow computing means automatically set a new reference point based on the reference point selection rule stored.

11. An ultrasonic treatment apparatus according to any one of claims 3 through 8, **characterized in that** the trace and follow computing means computes and estimates a moved position of the treatment region based on a blackened portion which occurs near the treatment region on the ultrasonic tomographic image in association with the advancement of the treatment use ultrasonic beams irradiation to the treatment region.

12. An ultrasonic treatment apparatus according to any one of claims 3 through 8, **characterized in that** the ultrasonic tomographic image taking means includes means for detecting a modulus of elasticity of the tissue in the region of interest, and the trace and follow computing means traces and detects a position of a region of which modulus of elasticity detected by the modulus of elasticity detecting means changes in association with advancement of the treatment use ultrasonic beams irradiation to the treatment region, and a moved position of the treatment region is computed and estimated based on the position of the modulus of elasticity changed region traced and detected.

13. An ultrasonic treatment apparatus according to any one of claims 3 through 8, **characterized in that** the trace and follow computing means respectively traces and detects the positions of the respective reference points by comparing the pixel value distribution pattern within a predetermined extent surrounding the respective reference points with the pixel value distribution pattern of the ultrasonic tomographic image.

14. An ultrasonic treatment apparatus according to any one of claims 2 through 13, **characterized in that** the trace and follow computing means controls the drive signal feeding means so as to stop irradiation of the treatment use ultrasonic beams when the movement amount of at least one reference point exceeds a predetermined threshold value.

15. An ultrasonic treatment apparatus according to claim 14, **characterized in that** the trace and follow computing means controls the drive signal feeding means so as to restart irradiation of the treatment use ultrasonic beams when the movement amount of at least one reference point stabilizes within a predetermined threshold value or when the position of at least one reference point returns within a predetermined extent of the original position.

16. An ultrasonic treatment apparatus according to any one of claims 3 through 8, **characterized in that** further comprising a temperature measuring means which measures temperature of the treatment region and a nearby healthy tissue, and the drive signal feeding means is controlled so as to stop irradiation of the treatment use ultrasonic beams when a temperature of the healthy tissue at least from the temperature measuring means exceeds a predetermined threshold value.

17. An ultrasonic treatment apparatus according to claim 16, **characterized in that** the drive signal feeding means is controlled so as to restart irradiation of the treatment use ultrasonic beams when a temperature of the healthy tissue at least from the temperature measuring means comes within a predetermined threshold value.

18. An ultrasonic treatment apparatus according to claim 17, **characterized in that** the drive signal feeding means is controlled to adjust irradiation interval of the treatment use ultrasonic beams so that a temperature of the healthy tissue from the temperature measuring means remains within a predetermined threshold value.

19. An ultrasonic treatment apparatus according to any one of claims 3 through 8, **characterized in that** the treatment region set on the ultrasonic tomographic image displayed on the displaying means is displayed by a first color and the reference point of the treatment use ultrasonic beams is displayed by a second color respectively.

20. An ultrasonic treatment apparatus according to claim 19, **characterized in that** the color of the first color is varied before and after a predetermined time irradiation of the treatment use ultrasonic beams.
